# FASCICULE DE BREVET EUROPEEN

(11) **EP 4 355 249 B1**
(45) Date de publication et mention de la délivrance du brevet: **29.01.2025**
(21) Numéro de dépôt: 22741336.6
(22) Date de dépôt: 14.06.2022
(51) Int. Cl.: A61B 34/20, A61B 34/32, A61B 90/00, A61B 34/10, A61B 34/30, A61B 17/00

(54) **ROBOT MÉDICAL POUR LE PLACEMENT D'INSTRUMENTS MÉDICAUX SOUS GUIDAGE ÉCHOGRAPHIQUE**
MEDIZINISCHER ROBOTER ZUR PLATZIERUNG MEDIZINISCHER INSTRUMENTE UNTER ULTRASCHALLFÜHRUNG
MEDICAL ROBOT FOR PLACEMENT OF MEDICAL INSTRUMENTS UNDER ULTRASOUND GUIDANCE

(30) Priorité: 16.06.2021 FR 2106350
(43) Date de publication de la demande: 24.04.2024
(73) Titulaire: Quantum Surgical, 34000 Montpellier (FR)
(72) Inventeur: SUDRE, Mathieu, 34160 Restinclières (FR); BLONDEL, Lucien, 34070 Montpellier (FR); NAHUM, Bertin, 34170 Castelnau-le-Lez (FR); BADANO, Fernand, 69006 Lyon (FR)
(74) Mandataire: Ipside
(86) Numéro de dépôt international: PCT/FR2022/051136
(87) Numéro de publication internationale: WO 2022/263763

(56) Documents cités:
- WO-A1-2021/099729
- US-A1- 2008 287 794
- US-A1- 2014 316 247
- US-A1- 2018 092 628
- US-B1- 6 546 279

## Description

### Domaine de l'invention

La présente demande appartient au domaine des dispositifs robotisés pour assister un praticien lors d'une intervention médicale mini-invasive comprenant l'insertion d'un ou plusieurs instruments médicaux dans une anatomie d'intérêt d'un patient. Notamment, l'invention concerne un robot médical configuré pour suivre le mouvement d'un point cible dans une lésion au sein d'une anatomie d'intérêt d'un patient et pour ajuster en temps réel la position d'un bras articulé du robot pour guider un instrument médical de façon optimale vers le point cible. Le mouvement du point cible peut notamment être engendré par la respiration du patient, ou par l'insertion de l'instrument médical.

### Etat de la technique

Pour préparer une intervention mini-invasive visant à atteindre avec un instrument médical une zone anatomique cible dans une anatomie d'intérêt d'un patient, un praticien effectue généralement une planification de l'intervention à partir d'une image médicale préopératoire (quelques jours ou quelques semaines avant l'intervention) ou pré-interventionnelle (juste avant l'intervention lorsque le patient est installé sur la table d'intervention). L'intervention médicale mini-invasive peut notamment viser à réaliser la biopsie ou l'ablation d'une tumeur dans un organe, à effectuer une vertébroplastie, une cimentoplastie ou encore à stimuler une zone anatomique particulière. L'anatomie d'intérêt peut correspondre par exemple à un poumon, un rein, le foie, le cerveau, le tibia, le genou, une vertèbre, etc. L'instrument médical peut être une aiguille, une sonde, un cathéter, etc.

Au cours de cette étape de planification, le praticien définit un point cible dans une région à traiter de l'anatomie d'intérêt. Le praticien définit également un point d'entrée pour l'instrument médical sur la peau du patient. Ces deux points définissent alors une trajectoire que l'instrument médical doit suivre pour réaliser l'intervention médicale. Dans le cas particulier des organes mous situés dans la région thoracique, la région abdominale ou la région pelvienne, les mouvements liés à la respiration du patient et/ou les déformations locales de l'organe dues à l'insertion de l'instrument médical entrainent un déplacement du point cible pendant l'intervention. Les images médicales de planification préopératoire ou pré-interventionnelle ne permettent pas de prévoir ce déplacement du point cible pendant l'intervention. Ainsi, la position du point cible (c'est-à-dire la position de la région à traiter dans l'anatomie d'intérêt) est généralement différente lors de l'acquisition de l'image médicale de planification et lors de l'intervention. Par conséquent, lorsque l'insertion de l'instrument médical est planifiée à partir de l'image médicale de planification, il y a un risque que le point cible ne soit pas atteint avec précision par l'instrument médical.

En outre, il existe un risque que l'instrument médical se courbe au cours de l'insertion et qu'il n'atteigne pas le point cible si la trajectoire planifiée que doit suivre l'instrument médical n'est pas ajustée en conséquence.

Pour limiter le déplacement du point cible engendré par la respiration du patient, il est envisageable, au moment de l'insertion de l'instrument médical, de bloquer la respiration du patient à une phase du cycle respiratoire correspondant à celle à laquelle l'image médicale de planification a été acquise. Le blocage de la respiration peut être effectué de façon volontaire par le patient si l'intervention médicale a lieu sous anesthésie locale, ou bien de façon contrôlée par le praticien si l'intervention médicale a lieu sous anesthésie générale (interruption de la ventilation mécanique). Cette solution n'est cependant pas toujours très précise car il est difficile d'obtenir une correspondance exacte entre la phase du cycle respiratoire à laquelle l'image médicale de planification a été acquise et la phase du cycle respiratoire à laquelle la respiration du patient est bloquée pendant l'intervention. En outre, cette solution suppose une insertion relativement rapide de l'instrument médical puisque celle-ci doit se faire pendant que la respiration du patient est bloquée.

Il est également envisageable de prendre plusieurs images médicales de planification au cours d'un cycle respiratoire du patient et de déterminer la trajectoire la moins soumise aux déformations et aux déplacements de l'anatomie d'intérêt engendrés par la respiration. Toutefois, il y a là encore un risque que le point cible ne soit pas atteint avec précision par l'instrument médical.

Il est également envisageable de suivre la position du point cible tout au long de l'intervention en faisant régulièrement l'acquisition d'images médicales intra-interventionnelles (images acquises lorsque l'instrument médical est inséré dans le corps du patient). Ces images médicales sont généralement acquises par tomodensitométrie, par rayons X ou par résonance magnétique. Dans le cas de la tomodensitométrie ou des rayons X, une telle solution présente toutefois l'inconvénient d'irradier significativement le patient et le praticien pendant l'intervention. Dans le cas de l'imagerie par résonance magnétique, il est nécessaire d'utiliser du matériel spécifique amagnétique, notamment pour le matériel anesthésique, ce qui est particulièrement contraignant. Cette solution suppose en outre l'utilisation de dispositifs d'imagerie encombrants tout au long de l'intervention.

Il est également connu de suivre la position d'une lésion au sein d'une anatomie d'intérêt à l'aide d'images échographiques. Toutefois, la lésion n'est pas toujours visible sur une image échographique, et les solutions existantes manquent généralement de précision.

Il reste donc nécessaire de trouver une solution pour insérer un instrument médical avec précision au niveau d'un point cible d'une région à traiter au sein d'une anatomie d'intérêt d'un patient, notamment lorsque les mouvements liés à la respiration du patient et/ou les déformations locales de l'anatomie d'intérêt dues à l'insertion de l'instrument médical entrainent un déplacement du point cible pendant l'intervention.

La demande de brevet US2008/287794A1 divulgue une méthode pour mettre en œuvre un système d'imagerie et de navigation pour effectuer une procédure médicale telle qu'une ablation cardiaque. La méthode comprend l'utilisation d'un dispositif d'imagerie ultrasonique pour fournir une image tridimensionnelle du patient en temps réel, l'identification d'un site cible sur l'image tridimensionnelle du patient en temps réel, l'orientation d'un instrument médical vers le site cible à l'aide d'un système de suivi et l'exécution de la procédure médicale sur le site cible.

La demande de brevet US2014/316247A1 divulgue une méthode et un appareil de suivi d'un changement dans une région d'intérêt chez un sujet en fonction de la respiration. Un « signal de respiration » est capturé à l'aide d'images échographiques. Il s'agit d'une représentation en fonction du temp du déplacement de la région d'intérêt.

La demande de brevet US2018/092628A1 décrit un appareil de diagnostic par ultrasons. Des données d'une image échographique acquise par une sonde à ultrasons sont associées à la position de la sonde. Un alignement d'image entre l'image échographique et une image médicale peut être réalisée.

### Exposé de l'invention

Les dispositifs et les méthodes divulgués dans la présente demande ont pour objectif de remédier à tout ou partie des inconvénients de l'art antérieur, notamment ceux exposés ci-avant.

A cet effet, et selon un premier aspect, il est proposé un robot médical pour assister un praticien lors d'une intervention médicale pour traiter une lésion dans une anatomie d'intérêt d'un patient. Le robot médical comporte un bras robotisé comportant à une extrémité distale un guide-outil destiné à guider un instrument médical. Le robot médical comporte également une unité de contrôle configurée pour contrôler le bras robotisé. Le robot médical est configuré pour coopérer avec un système de navigation et avec une sonde à ultrasons destinée à être positionnée par le praticien au niveau de l'anatomie d'intérêt du patient. L'unité de contrôle est configurée pour pouvoir déterminer en tout instant, à partir d'informations communiquées par le système de navigation, la position d'un marqueur robot destiné à être fixé sur le robot médical, la position d'un marqueur patient destiné à être positionné sur le patient à proximité de l'anatomie d'intérêt, et la position d'un marqueur sonde destiné à être fixé sur la sonde à ultrasons. Pendant une phase de modélisation, l'unité de contrôle est configurée pour recevoir une pluralité d'images échographiques acquises par la sonde à ultrasons pendant au moins un cycle respiratoire du patient et pour générer, à partir desdites images échographiques, un modèle permettant d'estimer la position d'un point cible au niveau de la lésion et la position d'un point d'entrée au niveau de la peau du patient, en fonction de la position du marqueur patient, quel que soit l'instant considéré dans un cycle respiratoire du patient. Pendant une phase de guidage, l'unité de contrôle est configurée pour contrôler en temps réel le bras robotisé en fonction de la position du marqueur patient pour guider l'instrument médical selon une trajectoire définie par la position du point cible et la position du point d'entrée associées à la position du marqueur patient dans le modèle.

Dans la présente demande, le terme « position » doit être entendu au sens large comme décrivant à la fois la position et l'orientation d'un objet dans un repère tridimensionnel (le terme « pose » est parfois utilisé dans la littérature anglo-saxonne). Les positions des marqueurs (marqueur patient, marqueur robot et marqueur sonde) ainsi que la position du point cible et la position du point d'entrée peuvent être définies dans un référentiel du robot ou dans un référentiel du système de navigation. Il convient de noter que le référentiel du robot peut être défini relativement au référentiel du système de navigation car la position du marqueur robot est connue à la fois dans le référentiel du système de navigation et dans le référentiel du robot (chaque articulation du bras robotisé comporte par exemple un codeur permettant de connaître la position de chaque élément articulé du bras robotisé dans le référentiel du robot, et la position du marqueur robot sur le robot est connue a priori par l'unité de contrôle).

La phase de modélisation a lieu au début de l'intervention, avant d'approcher le bras robotisé de l'anatomie d'intérêt et avant d'insérer l'instrument médical dans le corps du patient. La phase de modélisation permet de modéliser le mouvement du point cible et le mouvement du point d'entrée par rapport au marqueur patient au cours d'un cycle respiratoire. Pendant la phase de modélisation, les positions du point cible et les positions du point d'entrée sont corrélées aux positions du marqueur patient, il est ainsi possible de définir la position du point cible et la position du point d'entrée en fonction de la position du marqueur patient. Le modèle obtenu permet donc de définir, à n'importe quel instant du cycle respiratoire, la position du point cible et la position du point d'entrée qui correspondent à la trajectoire que doit suivre l'instrument médical pour atteindre avec précision le point cible.

Une fois que la position du point cible et la position du point d'entrée sont modélisées en fonction de la position du marqueur patient, il devient possible, en suivant en temps réel la position du marqueur patient à l'aide du système de navigation, de définir en temps réel la position que doit prendre le bras robotisé pour que l'instrument médical soit guidé selon la trajectoire définie par la position du point cible et la position du point d'entrée associées à la position du marqueur patient. Ce suivi en temps réel peut notamment avoir lieu avant l'insertion de l'instrument médical.

Avec de telles dispositions, il devient possible de bloquer la respiration du patient à n'importe quel instant du cycle respiratoire pour procéder à l'insertion de l'instrument médical. En effet, quel que soit l'instant où la respiration du patient est bloquée, le bras robotisé sera correctement positionné pour permettre l'insertion de l'instrument médical selon la trajectoire souhaitée.

Aussi, il n'est même plus indispensable de bloquer la respiration du patient pendant l'intervention. En effet, le bras robotisé est déplacé en temps réel pour que la position du bras robotisé soit constamment ajustée pour guider l'instrument médical selon la trajectoire souhaitée.

L'invention permet en outre de minimiser les réajustements latéraux de la trajectoire après l'insertion de l'instrument médical (de tels réajustements latéraux de la trajectoire sont généralement traumatiques pour l'organe traversé par l'instrument médical).

L'instrument médical peut ainsi être inséré au niveau de la région à traiter avec une très grande précision, quel que soit l'instant d'insertion de l'instrument médical au cours du cycle respiratoire. L'insertion de l'instrument médical est généralement mise en œuvre par le praticien, le robot médical ayant pour but de guider l'insertion de l'instrument médical par le praticien. Rien n'empêcherait toutefois que l'insertion de l'instrument médical soit automatisée et contrôlée par l'unité de contrôle.

En outre, comme la modélisation de la position du point cible et de la position du point d'entrée au cours d'un cycle respiratoire est réalisée à partir d'images échographiques, le patient et le praticien ne sont pas exposés à des rayonnements ionisants au cours de l'intervention.

Dans des modes particuliers de réalisation, l'invention peut comporter en outre l'une ou plusieurs des caractéristiques suivantes, prises isolément ou selon toutes les combinaisons techniquement possibles.

Dans des modes particuliers de réalisation, pendant la phase de modélisation, l'unité de contrôle est configurée, pour chaque image échographique reçue, pour :
- déterminer la position du marqueur patient et la position du marqueur sonde à l'instant auquel ladite image échographique a été acquise par la sonde à ultrasons,
- obtenir une image d'analyse, à partir de l'image échographique, sur laquelle la lésion est visible,
- déterminer, sur l'image d'analyse, un point cible au niveau de la lésion et un point d'entrée au niveau de la peau du patient, le point cible et le point d'entrée définissant ainsi une trajectoire à suivre pour l'instrument médical,
- déterminer la position du point cible et la position du point d'entrée à partir de la position du marqueur sonde,
- établir une association entre la position du marqueur patient, la position du point cible et la position du point d'entrée ainsi déterminées pour ladite image échographique.

En outre, l'unité de contrôle est configurée pour modéliser, à partir des informations ainsi obtenues pour la pluralité d'images échographiques, la position du point cible et la position du point d'entrée, en fonction de la position du marqueur patient, quel que soit l'instant considéré dans un cycle respiratoire du patient.

Dans des modes particuliers de réalisation, pendant la phase de guidage, lors d'une insertion de l'instrument médical, l'unité de contrôle est configurée pour recevoir régulièrement de nouvelles images échographiques acquises par la sonde à ultrasons. L'unité de contrôle est en outre configurée pour mettre à jour le modèle à partir desdites nouvelles images échographiques.

Dès que le praticien commence à insérer l'instrument médical dans le corps du patient, la position du point d'entrée au niveau de la peau du patient est fixe et devient un pivot de rotation pour les mouvements du bras robot. Il reste toutefois possible de suivre en temps réel la position du point cible, la position du point d'entrée, et la position du marqueur patient avec de nouvelles images échographiques acquises en temps réel pendant la phase d'insertion de l'instrument médical. De telles dispositions permettent de prendre en compte un éventuel déplacement du point cible résultant de l'insertion de l'instrument médical. Le point cible peut en effet se déplacer dans la direction de la trajectoire suivie par l'instrument médical au cours de son insertion (c'est notamment le cas lorsque le point cible à atteindre est dans une lésion, par exemple une tumeur, au sein d'un organe mou). La détermination en temps réel de la position du point cible à l'aide des images échographiques permettent de mettre à jour en temps réel la trajectoire que doit suivre l'instrument médical ainsi que la position du bras robotisé pour guider l'instrument médical selon cette trajectoire.

Dans des modes particuliers de réalisation, pendant la phase de guidage, lors de l'insertion de l'instrument médical, l'unité de contrôle est en outre configurée pour déterminer, pour chaque nouvelle image échographique reçue, la position de l'instrument médical, et pour ajuster le contrôle en temps réel du bras robotisé en fonction de la position de l'instrument médical.

De telles dispositions permettent de prendre en compte le risque que l'instrument médical se courbe au cours de l'insertion et d'ajuster le contrôle en temps réel du bras robotisé en conséquence (la trajectoire à suivre par l'instrument médical n'est alors plus une ligne droite entre le point d'entrée et le point cible).

Dans des modes particuliers de réalisation, l'image d'analyse correspond directement à l'image échographique. C'est le cas notamment lorsque la lésion est visible sur l'image échographique.

Dans des modes particuliers de réalisation, l'unité de contrôle est configurée pour obtenir l'image d'analyse en fusionnant l'image échographique avec une image de référence préopératoire ou pré-interventionnelle sur laquelle la lésion est visible.

En effet, lorsque la lésion n'est pas visible sur une image échographique (lésion isoéchogène), il n'est pas possible de déterminer la position du point cible directement sur l'image échographique. Il convient alors de recaler l'image échographique avec une image de référence de modalité différente sur laquelle la lésion est visible. Il peut s'agir notamment d'une image préopératoire ou pré-interventionnelle de tomodensitométrie, de tomographie par émission de positons ou d'imagerie par résonance magnétique. La fusion de l'image échographique avec l'image de référence donne alors une image d'analyse sur laquelle la lésion est visible. Le recalage peut être global (recalage sur la totalité de l'anatomie d'intérêt) ou local (recalage optimisé sur une zone particulière de l'anatomie d'intérêt). Le recalage peut être effectué de manière rigide (par translation et/ou rotation) ou de manière non-rigide (avec déformation). Le recalage peut notamment être mis en œuvre par un algorithme d'apprentissage automatique basé sur la reconnaissance de structures anatomiques particulières sur les images à fusionner.

Dans des modes particuliers de réalisation, un élément radio-opaque du marqueur patient est visible sur l'image de référence, et l'unité de contrôle est configurée pour fusionner l'image échographique avec l'image de référence par un recalage basé sur la position du marqueur patient relativement à la position du marqueur sonde à l'instant auquel l'image échographique a été acquise par la sonde à ultrasons.

Dans des modes particuliers de réalisation, l'image de référence est une image de tomodensitométrie, une image de tomographie par émission de positons ou une image d'imagerie par résonance magnétique.

Dans des modes particuliers de réalisation, les images échographiques reçues de la sonde à ultrasons sont des images échographiques en mode B.

Dans des modes particuliers de réalisation, l'unité de contrôle est configurée pour recevoir et traiter des images échographiques acquises par la sonde à ultrasons à une fréquence au moins égale à quinze images par seconde.

De telles dispositions permettent de garantir un suivi en temps réel de la position du point cible et par conséquent un ajustement en temps réel de la position du bras robotisé pour que l'instrument médical soit guidé selon la trajectoire souhaitée tout au long de l'intervention.

Dans des modes particuliers de réalisation, le robot médical comporte en outre une interface utilisateur comprenant un écran d'affichage permettant au praticien de visualiser les images d'analyse.

Dans des modes particuliers de réalisation, l'interface utilisateur comporte des moyens d'entrée permettant au praticien d'identifier sur une image d'analyse affichée sur l'écran d'affichage un point cible et/ou un point d'entrée et/ou une zone anatomique qui ne doit pas être traversée par l'instrument médical.

Le praticien peut ainsi planifier l'intervention, à l'aide de l'interface utilisateur, sur une image pré-interventionnelle correspondant à une image d'analyse associée à une première image échographique acquise par la sonde à ultrasons (il peut s'agir directement de l'image échographique si la lésion est visible sur l'image échographique ou, dans le cas contraire, d'une image de fusion résultant du recalage de l'image échographique avec une image préopératoire de modalité différente sur laquelle la lésion est visible). Le point cible et le point d'entrée définis par le praticien sur cette première image sont ensuite déterminés automatiquement par l'unité de contrôle sur les images d'analyse suivantes. Le suivi du point cible peut notamment être mis en œuvre par une méthode de suivi de mouvement dans plusieurs images échographiques successives, par une analyse de déformation du « speckle » ou par un algorithme d'intelligence artificielle (le « speckle » représente l'ensemble des petites tâches rapidement fluctuantes qui apparaissent dans la texture instantanée d'une image et qui lui donnent un aspect granuleux). Lorsque la lésion n'est pas visible sur l'image échographique, pour aider au suivi du point cible sur les images d'analyse, il est avantageux de suivre le mouvement d'une structure anatomique proche de la lésion visible sur les images échographiques (par exemple un vaisseau sanguin).

Dans des modes particuliers de réalisation, l'interface utilisateur comprend un dispositif de réalité augmentée permettant de superposer les images d'analyse avec des images réelles du corps du patient sur l'écran d'affichage

Le dispositif de réalité augmentée permet de superposer au corps du patient la lésion en mouvement et en trois dimensions ainsi que la progression de l'instrument médical au cours de son insertion. Il peut s'agir par exemple d'un écran positionné sur la table d'intervention au-dessus du patient, ou encore d'un masque, d'un casque ou de lunettes de réalité augmentée. Ce type d'affichage facilite la représentation spatiale de l'anatomie d'intérêt du patient par le praticien.

Dans des modes particuliers de réalisation, l'unité de contrôle est configurée pour comparer une image échographique avec une image de référence sur laquelle la lésion est visible, et pour donner une indication au praticien de la direction dans laquelle il convient de déplacer la sonde à ultrasons pour qu'une image échographique acquise par la sonde à ultrasons comporte une région anatomique dans laquelle se trouve la lésion.

L'image de référence est par exemple une image préopératoire ou pré-interventionnelle de tomodensitométrie, de tomographie par émission de positons ou d'imagerie par résonance magnétique. L'indication de la direction dans laquelle il convient de déplacer la sonde à ultrasons est par exemple signalée au praticien sur l'écran d'affichage de l'interface utilisateur. Selon d'autres exemples, l'indication de la direction dans laquelle il convient de déplacer la sonde à ultrasons peut être signalée au praticien par des signaux lumineux ou par des retours haptiques (vibrations) au niveau de la sonde à ultrasons, ou bien sur un affichage en réalité augmentée.

Selon un deuxième aspect, il est proposé un dispositif médical comportant un robot médical selon l'un quelconque des modes de réalisation décrits précédemment, ainsi qu'un système de navigation et une sonde à ultrasons destinés à coopérer avec le robot médical.

### Présentation des figures

L'invention sera mieux comprise à la lecture de la description suivante, donnée à titre d'exemple nullement limitatif, et faite en se référant aux figures 1 à 15 qui représentent :
[Fig. 1] une représentation schématique d'un dispositif médical selon l'invention comportant un robot médical, un système de navigation et une sonde,
[Fig. 2] une représentation schématique du bras robotisé du robot médical,
[Fig. 3] une représentation schématique du guide-outil destiné à être fixé à l'extrémité du bras robotisé,
[Fig. 4] une représentation du guide-outil mettant en évidence un dispositif de maintien d'un instrument médical à l'extrémité du guide-outil,
[Fig. 5] une représentation du guide-outil mettant en évidence le positionnement de l'instrument médical sur le guide-outil ainsi que des éléments détectables par un système de navigation formant un « marqueur robot »,
[Fig. 6] une représentation schématique d'un « marqueur patient » destiné à être positionné sur le patient à proximité de l'anatomie d'intérêt,
[Fig. 7] une représentation schématique d'un « marqueur sonde » destiné à être fixé sur la sonde à ultrasons,
[Fig. 8] une représentation schématique des principales étapes d'un procédé mis en œuvre par l'unité de contrôle pendant une phase de modélisation (dans le cas où la lésion est visible sur les images échographiques) puis pendant une phase de contrôle en temps réel du bras robotisé avant l'insertion de l'instrument médical,
[Fig. 9] une représentation schématique d'une interface utilisateur permettant au praticien d'identifier sur une image un point cible et/ou un point d'entrée et/ou une zone à risque, ainsi que des paramètres de traitement,
[Fig. 10] une représentation schématique d'une image préopératoire ou pré-interventionnelle (partie a) de la figure), une image échographique (partie b) de la figure), et une image de fusion résultant du recalage de l'image préopératoire ou pré-interventionnelle et de l'image échographique (partie c) de la figure),
[Fig. 11] une représentation schématique des principales étapes d'un procédé mis en œuvre par l'unité de contrôle pendant une phase de planification basée sur une image préopératoire ou pré-interventionnelle puis pendant la phase de modélisation (cas où la lésion n'est pas visible sur les images échographiques),
[Fig. 12] une représentation schématique d'un mouvement estimé du marqueur patient au cours d'un cycle de respiration du patient,
[Fig. 13] une représentation schématique du suivi de la position du marqueur patient au cours du temps,
[Fig. 14] une représentation schématique de l'ajustement en temps réel de la position du bras robotisé à partir la modélisation de la position du point cible en fonction de la position du marqueur patient,
[Fig. 15] une représentation schématique des principales étapes d'un procédé mis en œuvre par l'unité de contrôle pendant une phase d'insertion de l'instrument médical.

Dans ces figures, des références identiques d'une figure à une autre désignent des éléments identiques ou analogues. Pour des raisons de clarté, les éléments représentés ne sont pas nécessairement à une même échelle, sauf mention contraire.

### Description détaillée d'au moins un mode de réalisation de l'invention

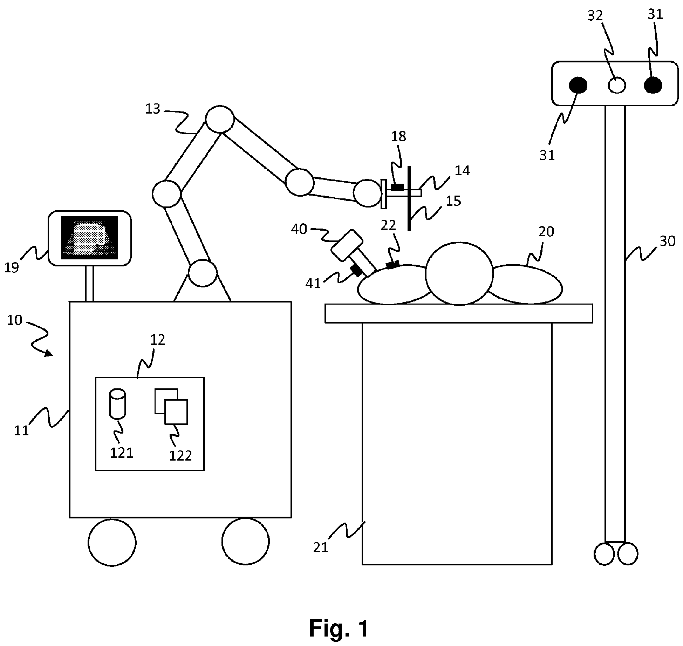

La figure 1 représente schématiquement un robot médical 10 selon l'invention. Le robot médical 10 est utilisé pour assister un praticien lors d'une intervention médicale sur une anatomie d'intérêt d'un patient 20 positionné sur une table d'intervention 21.

On se place à titre d'exemple dans le cas d'une intervention médicale effectuée par voie mini-invasive ou percutanée pour traiter une lésion au sein de l'anatomie d'intérêt du patient. Ce genre d'intervention nécessite généralement l'insertion par le praticien d'un ou plusieurs instruments médicaux (par exemple une aiguille, une sonde, un cathéter, etc.) dans le corps du patient jusqu'à une certaine profondeur pour atteindre une zone anatomique cible (une lésion, par exemple une tumeur) dans l'anatomie d'intérêt (par exemple dans le foie, un poumon, un rein, etc.).

Le robot médical 10 comporte une base 11. Dans l'exemple considéré, la base 11 du robot médical 10 est équipée de roues motorisées, ce qui permet au robot médical 10 de se déplacer selon différentes directions par des mouvements de translation et/ou de rotation.

Le robot médical 10 comporte en outre un bras robotisé 13 articulé dont une extrémité est reliée à la base 11. A l'autre extrémité du bras robotisé 13 est fixé un guide-outil 14 destiné à guider un instrument médical 15, comme par exemple une aiguille, une sonde, un cathéter, une électrode, etc. Le robot médical 10 peut alors être utilisé pour aider un praticien à positionner, maintenir, ou guider l'instrument médical 15 au cours de l'intervention médicale. Le robot médical 10 joue alors le rôle d'une troisième main pour le praticien.

Le robot médical 10 comporte une unité de contrôle 12 configurée pour contrôler le déplacement du bras robotisé 13. L'unité de contrôle 12 comporte par exemple un ou plusieurs processeurs 122 et une mémoire 121 (disque dur magnétique, mémoire électronique, disque optique, etc.) dans laquelle est mémorisée un produit programme d'ordinateur, sous la forme d'un ensemble d'instructions de code de programme à exécuter pour mettre en œuvre les différentes étapes d'un procédé de positionnement du bras robotisé 13. La mémoire 121 permet également d'enregistrer les images et autres informations (notamment les informations de navigation) utilisées pour mettre en œuvre ce procédé.

Le robot médical 10 peut également comporter une interface utilisateur 19 comprenant un écran d'affichage permettant au praticien de visualiser des images échographiques acquises par la sonde à ultrasons 40 ou d'autres images médicales (par exemple une image de référence préopératoire ou pré-interventionnelle de l'anatomie d'intérêt, ou bien des images de fusion issues du recalage des images échographiques avec une image de référence). L'interface utilisateur peut également comporter des moyens d'entrée (clavier, souris, écran tactile, etc.) permettant au praticien d'identifier sur une image affichée sur l'écran d'affichage un point cible et/ou un point d'entrée et/ou une zone anatomique qui ne doit pas être traversée par l'instrument médical 15.

Dans des modes particuliers de réalisation, l'interface utilisateur peut comprendre un dispositif de réalité augmentée permettant de superposer les images échographiques (ou les images de fusion) avec des images réelles du corps du patient sur l'écran d'affichage. Un tel dispositif facilite la représentation spatiale de l'anatomie d'intérêt pour le praticien.

Le robot médical 10 est configuré pour coopérer avec un système de navigation 30 et avec une sonde à ultrasons 40 destinée à être positionnée par le praticien au niveau de l'anatomie d'intérêt du patient. Le robot médical 10 comporte un module de communication connecté à l'unité de contrôle 12 pour échanger des données avec le système de navigation 30 et avec la sonde à ultrasons 40. Le système de navigation 30 et la sonde à ultrasons 40 comportent également chacun un module de communication pour échanger des données avec l'unité de contrôle 12 du robot médical 10. Les communications établies entre l'unité de contrôle 12, le système de navigation 30 et la sonde à ultrasons peuvent être des communications filaires ou des communications sans fil. Par souci de simplification, les modules de communication ne sont pas représentés sur la figure 1.

Dans l'exemple considéré, le système de navigation 30 est un système de navigation optique. Le système de navigation 30 comporte deux capteurs optiques 31 correspondant à deux capteurs d'une caméra stéréoscopique fonctionnant dans le domaine des rayonnements infrarouges. Dans l'exemple considéré, le système de navigation 30 comporte en outre une caméra 32 fonctionnant dans le domaine de la lumière visible.

L'unité de contrôle 12 est configurée pour pouvoir déterminer en tout instant, à partir d'informations communiquées par le système de navigation 30, la position d'un marqueur robot 18 destiné à être fixé sur le robot médical 10, la position d'un marqueur patient 22 destiné à être positionné sur le patient 20 à proximité de l'anatomie d'intérêt, et la position d'un marqueur sonde 41 destiné à être fixé sur la sonde à ultrasons 40.

Dans la présente demande, le terme « position » correspond à la combinaison de la position et de l'orientation d'un objet dans un repère donné qui est généralement un système de coordonnées en trois dimensions. Le terme « pose » est employé dans la littérature anglo-saxonne pour représenter cette combinaison de la position et de l'orientation d'un objet dans l'espace.

L'unité de contrôle 12 est configurée pour recevoir de la part de la sonde à ultrasons 40 des images échographiques acquises par la sonde à ultrasons 40.

Les images échographiques reçues en provenance de la sonde à ultrasons 40 et les informations reçues en provenance du système de navigation 30 sont synchronisées temporellement par l'unité de contrôle 12 pour pouvoir corréler à un instant donné la position de la lésion avec la position du marqueur patient 22.

De façon conventionnelle, la sonde à ultrasons 40 comprend un ou plusieurs éléments émetteur-récepteur d'ondes sonores (matériaux piézo-électriques, transducteurs électroniques capacitifs). La sonde à ultrasons produit des ondes ultrasonores par effet piézo-électrique indirect. Chaque fois qu'une onde rencontre une structure anatomique, une partie de cette onde revient par réflexion ou diffusion (« speckle ») sous la forme d'un écho. Cet écho est ensuite transformé en courant électrique par effet piézo-électrique direct puis reconstruit en image. La reconstruction d'une image échographique dépend principalement du nombre, de la taille et des positions des éléments émetteur-récepteur de la sonde (résolution latérale et longitudinale), de la durée des pulses d'émission et des temps d'écho (résolution axiale et/ou en profondeur). L'énergie de l'écho réceptionnée est ensuite codée en niveau de gris. Plus l'énergie est haute et plus la portion d'image correspondante (pixel) est blanche. Ce codage en nuance de gris est appelé la « brillance » et le mode échographique associé est appelé « mode-B ». Les images produites par la sonde à ultrasons 40 peuvent être des images en deux dimensions ou des images en trois dimensions. De préférence, la sonde à ultrasons 40 est capable de générer des images à une fréquence au moins égale à quinze images par seconde.

Le mode-B est particulièrement bien adapté lorsque l'anatomie d'intérêt est le foie. Il convient toutefois de noter que l'invention pourrait également s'appliquer avec d'autres modes échographiques, comme par exemple l'élastographie.

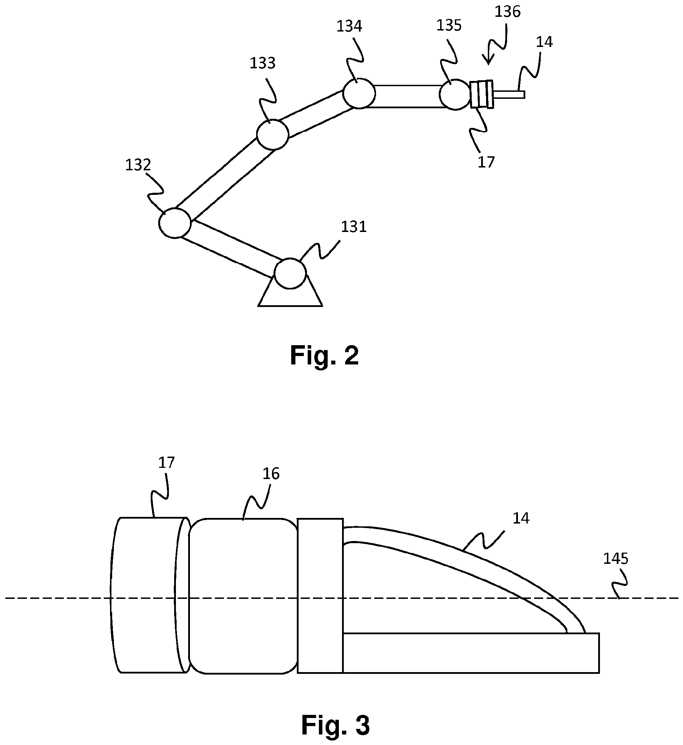

Dans l'exemple considéré, et tel qu'illustré sur la figure 2, le bras robotisé 13 comporte six articulations rotoïdes 131 à 136 conférant six degrés de liberté permettant de positionner et/ou déplacer l'instrument médical 15 dans n'importe quelle position de l'espace tri-dimensionnel. Avantageusement, les articulations 131 à 135 du bras robotisé 13 ne sont pas alignées et présentent un décalage les unes par rapport aux autres, ce qui permet un plus grand nombre de configurations possibles du bras robotisé 13. Chaque articulation comprend au moins un codeur permettant de connaître en temps réel sa position angulaire. Une configuration du bras robotisé 13 correspond alors à un ensemble de valeurs de paramètres prises par les articulations 131 à 136 (par exemple la valeur d'un angle de rotation pour chaque articulation). L'articulation rotoïde 136 correspond à une rotation autour de l'axe principal du guide-outil 14. Il convient de noter qu'il n'est toutefois pas nécessaire d'effectuer une rotation autour de l'axe de symétrie de l'instrument médical (cinq degrés de liberté sont en effet suffisants pour guider et relâcher un instrument médical). Ce degré de liberté supplémentaire permet d'être en situation de redondance et d'avoir une infinité de configurations possibles du bras robotisé 13 pour une position donnée du guide-outil 14. Cette situation de redondance est particulièrement utile pour s'adapter à des contraintes liées à la position du patient ou à la configuration de la salle d'intervention. Cette situation de redondance permet notamment de s'adapter à l'enveloppe externe du patient et à la position des marqueurs, par exemple si une configuration du bras robotisé cache un des marqueurs, il est possible d'adopter une autre configuration du bras robotisé 13 tout en conservant une même trajectoire pour l'instrument médical 15.

Dans l'exemple considéré, et tel qu'illustré sur la figure 3, le guide-outil 14 est fixé sur le bras robotisé 13 par l'intermédiaire d'une bride 17. Le guide-outil comporte un axe principal 145 représenté sur la figure 3 par une ligne en pointillé. Le guide-outil 14 est couplé à un capteur de force 16 pour permettre à l'unité de contrôle 12 de déterminer une force exercée sur le guide-outil 14. Cette force peut notamment être exercée par le praticien lorsqu'il déplace manuellement le bras robotisé 13. Cette force peut également correspondre à une force exercée sur le guide-outil 14 via l'instrument médical 15 par le corps du patient.

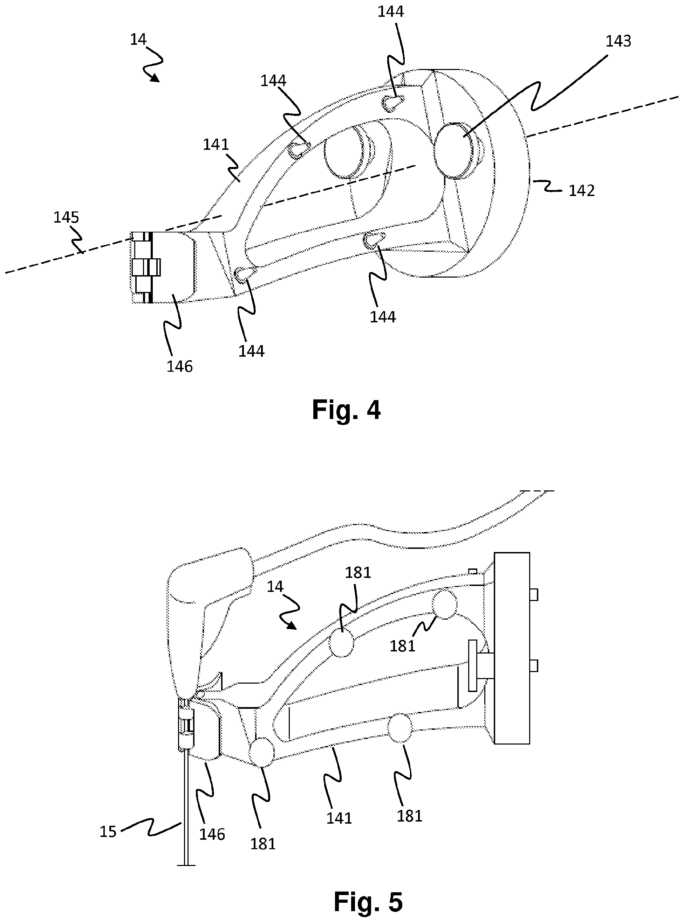

Dans l'exemple considéré, et tel qu'illustré sur les figures 4 et 5, le guide-outil 14 comporte un corps 141 avec une base 142 destinée à être fixée à la bride 17 à l'aide de vis 143, ainsi qu'un système de maintien 146 comportant deux parties mobiles l'une par rapport à l'autre. Le système de maintien 146 est destiné à maintenir l'instrument médical 15 au niveau de l'extrémité du corps 141 du guide-outil 14 opposée à la base 142. Les deux parties mobiles du système de maintien 146 peuvent être entrainées par un système d'entrainement tel qu'un engrenage, une came, une vis à filets inversés et/ou un actionneur linéaire, afin de bloquer ou libérer l'instrument médical 15. L'actionneur linéaire peut être réversible (le système de maintien 146 du guide-outil 14 peut alors être ouvert manuellement ou automatiquement sur commande de l'unité de contrôle 12) ou non réversible (le système de maintien 146 du guide-outil 14 ne peut être ouvert que de façon automatique sur commande de l'unité de contrôle). Le guide-outil 14 permet par exemple de guider des instruments médicaux de différents diamètres. Par exemple, un tel guide permet de guider des instruments médicaux dont le diamètre est compris entre 11 et 21 gauges. La gauge est une unité de mesure couramment utilisée pour définir le diamètre externe d'un instrument médical telle qu'une aiguille, une sonde ou un cathéter (11 gauges correspondent à un diamètre externe de 2.946 mm ; 21 gauges correspondent à un diamètre externe de 0.812 mm).

Tel qu'illustré sur les figures 4 et 5, le guide-outil 14 comporte des plots 144 destinés à accueillir des marqueurs optiques 181. Avantageusement, le guide-outil 14 comporte au moins trois marqueurs optiques 181 de telle sorte que la position du guide-outil 14 puisse être déterminée dans les trois dimensions spatiales du référentiel du système de navigation 30. Les positions respectives des marqueurs optiques 181 du guide-outil 14 les uns par rapport aux autres sont connues a priori par le dispositif de navigation 30 et/ou par l'unité de contrôle 12. Avantageusement, la forme géométrique de chaque marqueur optique 181 peut également être connue a priori. Dans l'exemple illustré à la figure 5, les marqueurs optiques 181 sont de forme sphérique.

L'ensemble des marqueurs optiques 181 présents sur le guide-outil 14 correspondent au marqueur robot 18.

L'utilisation d'au moins trois marqueurs optiques 181 permet de définir un plan et donc un repère tridimensionnel orthonormé direct avec un axe z normal au plan et des axes x et y dans le plan de façon que le repère soit direct. Cela permet ainsi de déterminer la position et l'orientation du repère formé à partir des marqueurs optiques 181 qui représentent le guide-outil 14. Les trois axes x, y et z permettent de définir six degrés de liberté, à savoir une translation selon chacun des axes x, y ou z et une rotation autour de chacun de ces axes.

Les marqueurs optiques 181 peuvent être passifs ou actifs. Des marqueurs optiques passifs réfléchissent un rayonnement optique émis par un autre élément, comme par exemple le système de navigation 30. Des marqueurs optiques passifs peuvent correspondre par exemple à des sphères réfléchissantes détectables par une caméra stéréoscopique infrarouge (c'est ce qui est utilisé par exemple dans les systèmes de navigation Polaris^{®} fabriqués par la société Northern Digital Inc.), ou à des motifs noir et blanc visibles par une caméra stéréoscopique (c'est ce qui est utilisé par exemple dans le système de navigation MicronTracker^{®} de la société ClaroNav). Des marqueurs optiques actifs émettent eux-mêmes un rayonnement optique, par exemple un rayonnement infrarouge, détectable par le système de navigation 30.

Il convient toutefois de noter qu'un seul marqueur optique présentant une forme géométrique caractéristique en trois dimensions pourrait être utilisé à la place de l'ensemble des marqueurs optiques 181 sphériques.

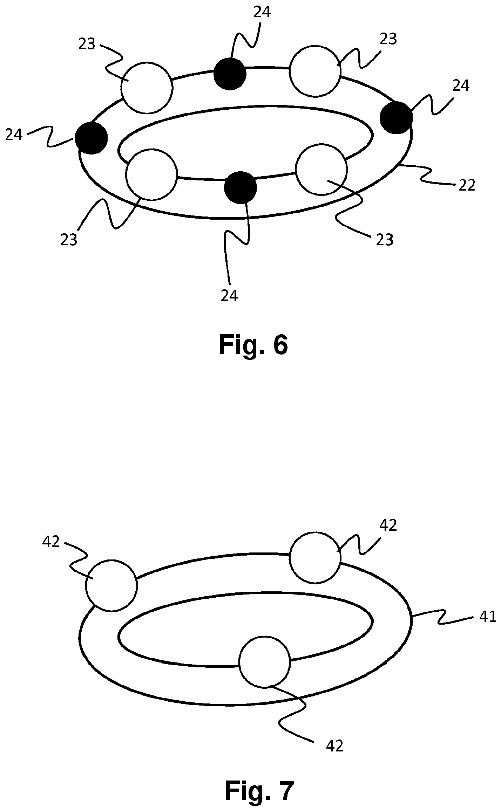

La figure 6 représente schématiquement le marqueur patient 22 destiné à être positionné sur le patient 20 à proximité de l'anatomie d'intérêt. Dans l'exemple considéré, le marqueur patient 22 comporte quatre marqueurs optiques 23, de telle sorte que la position du marqueur patient 22 puisse être déterminée dans les trois dimensions spatiales du référentiel du système de navigation 30. Les positions respectives des marqueurs optiques 23 du marqueur patient 22 les uns par rapport aux autres sont connues a priori par le système de navigation 30 et/ou par l'unité de contrôle 12. Avantageusement, la forme géométrique de chaque marqueur optique 23 peut également être connue a priori. Dans l'exemple illustré à la figure 6, les marqueurs optiques 23 sont de forme sphérique. La forme sphérique permet d'optimiser la réflexion du rayonnement optique. Ce qui a été mentionné précédemment pour le type actif ou passif des marqueurs optiques 181 du guide-outil 14 est également vrai pour les marqueurs optiques 23 de la référence patient 22. Là encore, il serait envisageable d'utiliser un seul marqueur optique présentant une forme géométrique caractéristique en trois dimensions à la place des quatre marqueurs optiques 23 sphériques.

Optionnellement, et tel qu'illustré sur la figure 6, le marqueur patient 22 peut également comporter des marqueurs radio-opaques 24 qui sont visibles sur une image médicale acquise par un dispositif d'imagerie médicale (par exemple par tomodensitométrie, par résonance magnétique, par ultrasons, par tomographie, par émission de positons, etc.). Les positions respectives des marqueurs radio-opaques 24 les uns par rapport aux autres sont connues a priori par le dispositif de navigation 30 et/ou par l'unité de contrôle 12. Avantageusement, la forme géométrique des marqueurs radio-opaques 24 peut également être connue a priori. Dans l'exemple considéré, le marqueur patient 22 comporte quatre marqueurs radio-opaques 24. Les marqueurs radio-opaques 24 peuvent être par exemple des billes en céramique. Il convient toutefois de noter qu'un unique marqueur radio-opaque présentant une forme géométrique caractéristique en trois dimensions pourrait être utilisé à la place des quatre marqueurs radio-opaques 24 sphériques.

La figure 7 représente schématiquement le marqueur sonde 41 destiné à être fixé sur la sonde à ultrasons 40 pour permettre au système de navigation 30 de déterminer la position de la sonde à ultrasons 40. Dans l'exemple considéré, le marqueur sonde 40 comporte trois marqueurs optiques 42, de telle sorte que la position du marqueur sonde 40 puisse être déterminée dans les trois dimensions spatiales du référentiel du système de navigation 30. Les positions respectives des marqueurs optiques 42 du marqueur sonde 40 les uns par rapport aux autres sont connues a priori par le système de navigation 30 et/ou par l'unité de contrôle 12. Avantageusement, la forme géométrique de chaque marqueur optique 42 peut également être connue a priori. Dans l'exemple illustré à la figure 7, les marqueurs optiques 42 sont de forme sphérique. La forme sphérique permet d'optimiser la réflexion du rayonnement optique. Ce qui a été mentionné précédemment pour le type actif ou passif des marqueurs optiques 181 du guide-outil 14 est également vrai pour les marqueurs optiques 42 de la référence sonde 40. Là encore, il serait envisageable d'utiliser un seul marqueur optique présentant une forme géométrique caractéristique en trois dimensions à la place des trois marqueurs optiques 42 sphériques.

Dans la suite de la description, on considère à titre d'exemple nullement limitatif que les capteurs optiques 31 du système de navigation 30 et les différents marqueurs optiques 181, 23, 42 sont conçus pour fonctionner avec un rayonnement optique de type infrarouge. On considère en outre que les marqueurs optiques 181, 23, 42 sont des marqueurs passifs. Les capteurs optiques 31 sont configurés pour émettre un rayonnement infrarouge. Ce rayonnement infrarouge est réfléchi par les différents marqueurs optiques 181, 23, 42 vers les capteurs optiques 31. Les capteurs optiques 31 sont configurés pour recevoir ce rayonnement infrarouge réfléchi. Le système de navigation 30 peut alors déterminer la distance entre un marqueur optique 181, 23, 42 et un capteur optique 31 en mesurant le temps pris par un rayon infrarouge pour faire le trajet aller-retour entre ledit capteur optique 31 et ledit marqueur optique 181, 23, 42. En connaissant la distance entre chaque marqueur optique 181, 23, 42 et chaque capteur optique 31, et en connaissant a priori l'agencement des marqueurs optiques 181, 23, 42 les uns par rapport aux autres sur le marqueur robot 18, sur le marqueur patient 22, et sur le marqueur sonde 41, il est possible de déterminer la position du marqueur robot 18, du marqueur patient 22, et du marqueur sonde 41 dans le référentiel du système de navigation 30. Il est à noter que la navigation optique par infrarouge est une méthode bien connue dans le domaine des interventions chirurgicales assistées par un robot médical.

Il convient de noter que l'invention est décrite en utilisant un système de navigation optique. Rien n'empêcherait toutefois d'utiliser, dans une variante, un système de navigation électromagnétique à la place du système de navigation optique. Dans ce cas, les différents « marqueurs » détectables par le système de navigation (marqueur patient 22, marqueur robot 18, et marqueur sonde 41) correspondraient alors à des capteurs électromagnétiques dont la position peut être déterminée par le système de navigation dans un champ électromagnétique généré.

Dans l'exemple considéré, l'unité de contrôle 12 du robot médical 10 est configuré pour recevoir du système de navigation 30 des informations sur la position courante du marqueur robot 18 dans le référentiel du système de navigation 30. Or, l'unité de contrôle 12 du robot médical 10 connaît la position courante du marqueur robot 18 dans le référentiel du robot médical 10 (via les codeurs des articulations 131 à 136). L'unité de contrôle 12 peut donc déterminer la transformation à opérer pour définir une position dans le référentiel du robot médical 10 à partir d'une position dans le référentiel du dispositif de navigation 30.

L'unité de contrôle 12 est également configurée pour recevoir du système de navigation 30 des informations sur la position du marqueur patient 22 et sur la position du marqueur sonde 41 dans le référentiel du système de navigation 30. L'unité de contrôle 10 peut alors définir la position du marqueur patient 22 et la position du marqueur sonde 41 dans le référentiel du robot médical 10.

Lorsque la position du marqueur sonde 41 est connue à un instant donné, il est possible de déterminer la position d'un élément visible sur une image échographique acquise par la sonde à ultrasons 40 audit instant. Cet élément visible peut être notamment un point cible à atteindre au niveau de la lésion à traiter ou un point d'entrée de l'instrument médical au niveau de la peau du patient. Le point cible et le point d'entrée définissent une trajectoire que doit suivre l'instrument médical 15. Lorsque la position du point cible et la position du point d'entrée sont connues, c'est-à-dire lorsque la trajectoire que doit suivre l'instrument médical 15 est définie, l'unité de contrôle peut déplacer automatiquement le bras robotisé 13 dans une configuration permettant au guide-outil 14 de guider l'instrument médical 15 selon la trajectoire définie.

Toutefois, et comme expliqué précédemment, les mouvements liés à la respiration du patient peuvent entrainer un déplacement du point cible. Ainsi, la trajectoire que doit suivre l'instrument médical 15 à un instant donné du cycle respiratoire du patient n'est pas la même à un autre instant du cycle respiratoire.

Pour résoudre ce problème, l'unité de contrôle 12 est configurée pour modéliser, pendant une phase de modélisation qui précède le geste chirurgical, le mouvement du marqueur patient 22 au cours d'au moins un cycle respiratoire du patient 20. Pendant la phase de modélisation, la position du point cible et la position du point d'entrée sont corrélées à la position du marqueur patient 22. Il est ainsi possible de définir la position du point cible et la position du point d'entrée en fonction de la position du marqueur patient. La modélisation obtenue permet donc de définir, à partir de la position du marqueur patient 22, et à n'importe quel instant du cycle respiratoire, la position du point cible et la position du point d'entrée qui correspondent à la trajectoire que doit suivre l'instrument médical 15 pour atteindre avec précision le point cible.

Une fois que la position du point cible et la position du point d'entrée sont modélisées en fonction de la position du marqueur patient 22, il devient possible, en suivant en temps réel la position du marqueur patient 22 à l'aide du système de navigation 30, de définir en temps réel la position que doit prendre le bras robotisé 13 pour que l'instrument médical 15 soit guidé selon la trajectoire définie par la position du point cible et la position du point d'entrée associées à la position du marqueur patient 22. Ce suivi en temps réel peut notamment avoir lieu pendant une phase de guidage, avant l'insertion de l'instrument médical 15. Le bras robotisé 13 est déplacé en temps réel pour que la position du bras robotisé soit constamment ajustée pour guider l'instrument médical selon la trajectoire souhaitée.

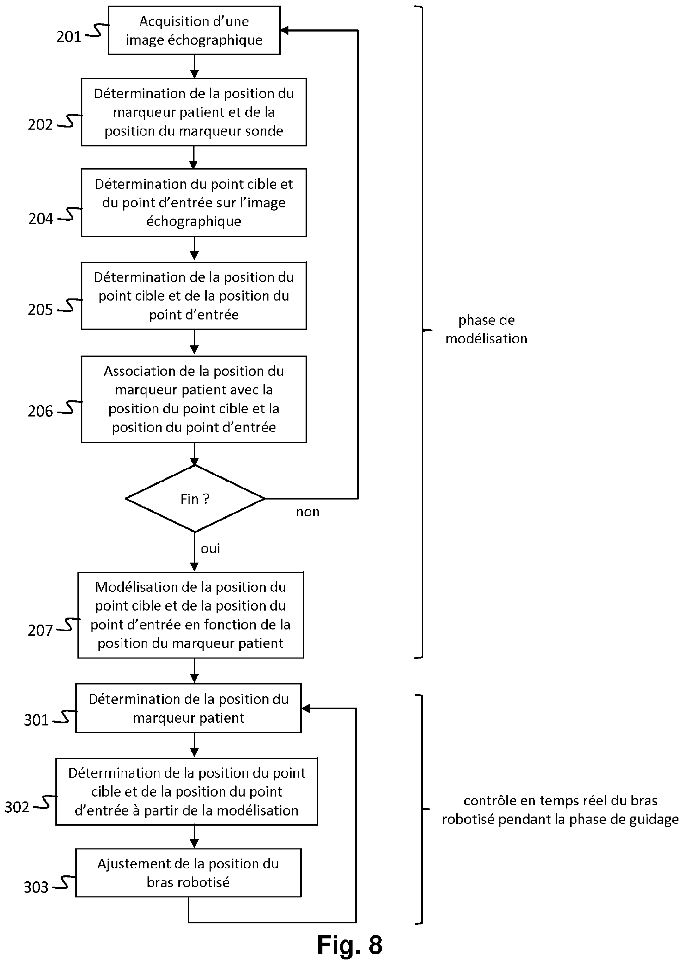

La figure 8 représente schématiquement les principales étapes d'un procédé mis en œuvre par l'unité de contrôle 12 pendant une phase de modélisation, puis pendant une phase de contrôle en temps réel du bras robotisé, avant l'insertion de l'instrument médical 15.

Pendant la phase de modélisation, l'unité de contrôle 12 est configurée pour recevoir une pluralité d'images échographiques acquises par la sonde à ultrasons 40 pendant au moins un cycle respiratoire du patient 20. Les étapes suivantes sont donc répétées tout au long de la phase de modélisation :
- acquisition 201 d'une image échographique (l'image échographique correspond à une image d'analyse sur laquelle la lésion est visible),
- détermination 202 de la position du marqueur patient 22 et de la position du marqueur sonde 41 à l'instant auquel ladite image échographique a été acquise par la sonde à ultrasons 40,
- détermination 204, sur l'image d'analyse, d'un point cible au niveau de la lésion et d'un point d'entrée au niveau de la peau du patient 20 (le point cible et le point d'entrée définissent une trajectoire à suivre pour l'instrument médical 15),
- détermination 205 de la position du point cible et la position du point d'entrée à partir de la position du marqueur sonde 41,
- association 206 entre la position du marqueur patient 22, la position du point cible et la position du point d'entrée ainsi déterminées pour ladite image échographique.

A la fin de la phase de modélisation, l'unité de contrôle 12 est configurée pour réaliser, à partir des informations ainsi obtenues pour les différentes images échographiques, une modélisation 207 de la position du point cible et de la position du point d'entrée, en fonction de la position du marqueur patient 22, quel que soit l'instant considéré dans un cycle respiratoire du patient.

Ensuite, pendant la phase de guidage, l'unité de contrôle 12 est configurée pour contrôler en temps réel le bras robotisé 13. Pour cela, à chaque instant, les étapes suivantes sont effectuées par l'unité de contrôle 12 :
- détermination 301 de la position du marqueur patient 22,
- détermination 302, à l'aide de la modélisation, de la position du point cible et la position du point d'entrée associées à la position du marqueur patient 22,
- déplacement 303 du bras robotisé 13 pour ajuster la position du guide-outil 14 de sorte que l'instrument médical 15 soit guidé par le guide-outil 14 selon la trajectoire définie par la position du point cible et la position du point d'entrée ainsi déterminées.

Une première détermination d'un point cible au niveau de la lésion et d'un point d'entrée au niveau de la peau du patient est par exemple initialement réalisée sur une première image d'analyse. Puis, par la suite, un algorithme de suivi de mouvement dans plusieurs images d'analyse successives peut être mis en œuvre pour déterminer le point cible et le point d'entrée sur chaque nouvelle image d'analyse, par exemple à l'aide d'une analyse de déformation du « speckle » (ensemble de tâches fluctuantes sur les images en raison de la diffusion des ondes) ou par un algorithme d'intelligence artificielle.

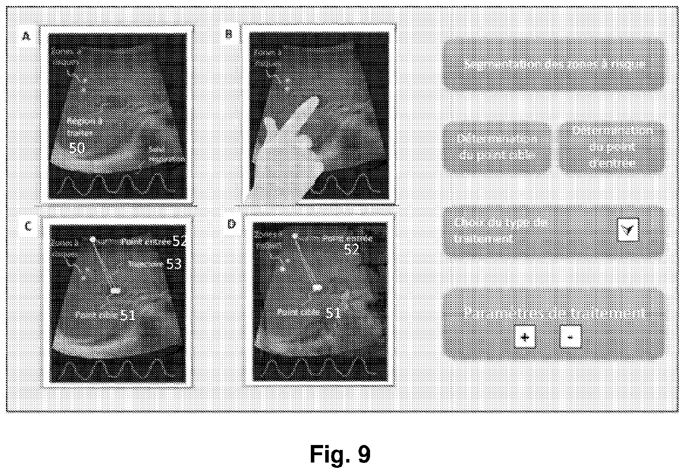

La première détermination d'un point cible et d'un point d'entrée peut être effectuée par le praticien à l'aide de l'interface graphique 19. La figure 9 illustre une interface utilisateur permettant au praticien d'identifier sur une image d'analyse un point cible 51 au niveau de la région à traiter 50, et/ou un point d'entrée 52 au niveau de la peau du patient, et/ou une zone à risque à éviter (par exemple les os ou les vaisseaux sanguins), ainsi que des paramètres de traitement. Cette étape peut être facilitée par une segmentation de certaines régions anatomiques (l'anatomie d'intérêt, la lésion à traiter, des zones à risque, etc.) par un algorithme d'apprentissage automatique.

Alternativement, la première détermination d'un point cible et d'un point d'entrée peut être mise en œuvre de façon automatique par un algorithme d'intelligence artificielle.

Il est toutefois possible que la lésion à traiter ne soit pas visible sur une image échographique, par exemple parce que la nature de la lésion fait qu'elle n'est pas (ou qu'elle est très peu) visible sur une image échographique. Dans un tel cas, il convient d'utiliser une image de référence pré-interventionnelle (c'est-à-dire une image acquise juste avant ou au début de l'intervention) ou préopératoire (c'est-à-dire une image acquise plusieurs jours ou plusieurs semaines avant l'intervention) sur laquelle la lésion est visible. Il peut notamment s'agir d'une image de tomodensitométrie, une image de tomographie par émission de positons ou une image d'imagerie par résonance magnétique. L'image de référence peut être une image en deux dimensions ou une image en trois dimensions. La première détermination d'un point cible et d'un point d'entrée peut alors être effectuée sur l'image de référence au lieu d'être effectuée sur une première image échographique. Là encore, la première détermination d'un point cible et d'un point d'entrée peut être effectuée par le praticien (par exemple à l'aide de l'interface graphique 19 illustrée sur la figure 9) ou bien de façon automatique par un algorithme d'intelligence artificielle. Une image échographique acquise par la sonde à ultrasons 40 peut alors ensuite être recalée avec l'image de référence pour former une image d'analyse correspondant à la fusion de l'image de référence avec l'image échographique. Le point cible et le point d'entrée sont alors visibles sur l'image d'analyse.

Alternativement, la première détermination d'un point cible et d'un point d'entrée peut être effectuée sur une première image d'analyse au lieu d'être effectuée sur l'image de référence.

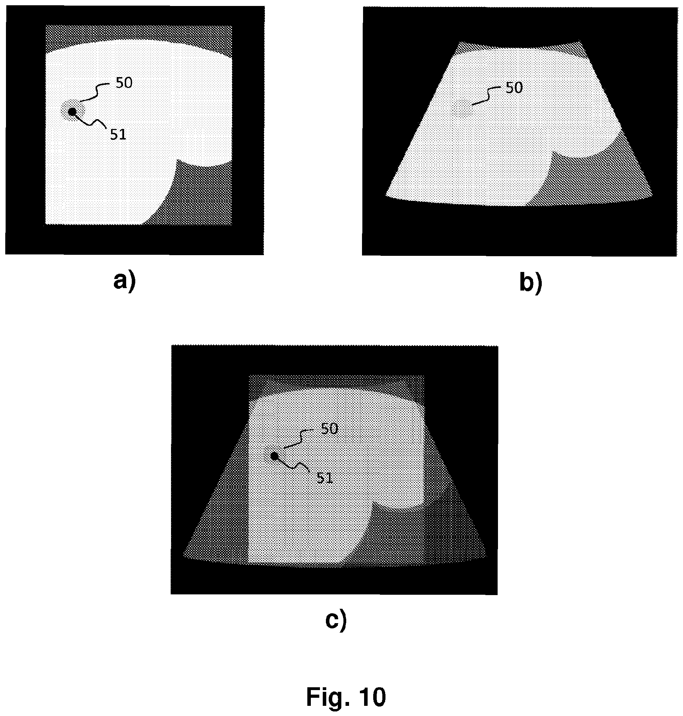

La figure 10 illustre schématiquement l'utilisation d'une image de référence préopératoire ou pré-interventionnelle (partie a) de la figure 10) pour recaler une image échographique (partie b) de la figure 10) afin de former une image d'analyse (partie c) de la figure 10) résultant du recalage et de la fusion de l'image de référence avec l'image échographique. La lésion à traiter 50 et le point cible 51 sont visibles sur l'image de référence. Dans l'exemple considéré, l'image de référence est acquise par tomodensitométrie. La lésion à traiter 50 n'est en revanche quasiment pas visible sur l'image échographique. La lésion à traiter 50 et le point cible 51 deviennent visibles sur l'image d'analyse issue du recalage de l'image de référence avec l'image échographique.

Le recalage est par exemple mis en œuvre par l'unité de contrôle 12 à l'aide d'un algorithme d'apprentissage automatique entrainé pour recaler globalement (recalage sur la totalité de l'anatomie) ou localement (optimisé sur une zone d'intérêt), de manière rigide (translation et rotation) ou non-rigide (déformation) une image tomodensitométrique d'une anatomie d'intérêt avec une image échographique. L'image résultant de ce recalage est appelée image d'analyse ou image de fusion.

Si des éléments radio-opaques du marqueur patient 22 sont visibles sur l'image de référence, un recalage rigide peut également être basé sur la position du marqueur patient 22 relativement à la position du marqueur sonde 41 à l'instant auquel l'image échographique a été acquise par la sonde à ultrasons 40.

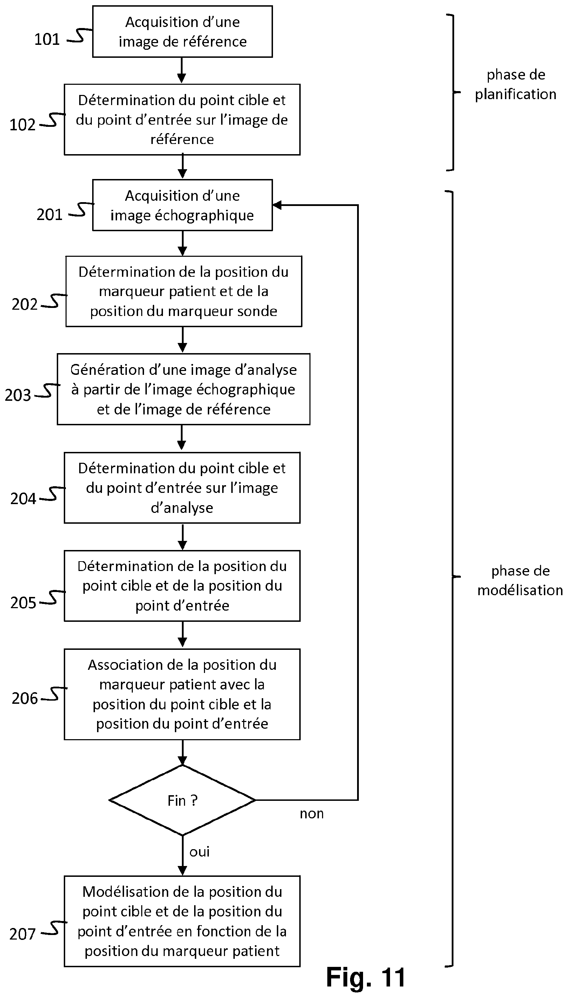

La figure 11 représente schématiquement les principales étapes mises en œuvre pendant une phase de planification basée sur une image de référence préopératoire ou pré-interventionnelle, puis pendant la phase de modélisation, dans le cas où la lésion n'est pas visible sur les images échographiques.

La phase de planification comporte notamment une étape d'acquisition 101 d'une image de référence préopératoire ou pré-interventionnelle puis une étape 102 de détermination du point cible 51 et du point d'entrée 52 sur l'image de référence.

La phase de modélisation comporte sensiblement les mêmes étapes que celles décrites en référence à la figure 8. Pour chaque image échographique reçue pendant la phase de modélisation, l'unité de contrôle 12 est en outre configurée pour mettre en œuvre une étape de génération 203 d'une image d'analyse par un recalage de l'image échographique avec l'image de référence. La détermination 204 du point cible et du point d'entrée est alors réalisée sur cette image d'analyse issue du recalage de l'image échographique avec l'image de référence (alors que dans le cas de la figure 8 l'image d'analyse correspond directement à l'image échographique).

Lorsque la lésion n'est pas visible sur l'image échographique, pour aider au suivi du point cible sur les images d'analyse, il peut être avantageux de suivre le mouvement d'une structure anatomique qui est proche de la lésion et qui visible sur les images échographiques (par exemple un vaisseau sanguin).

Il convient de noter que l'étape 102 peut être facultative si la première détermination du point cible et du point d'entrée est effectuée sur une première image d'analyse au lieu d'être effectuée sur l'image de référence.

Une fois le point cible 51 et le point d'entrée 52 déterminés sur une image d'analyse, leur position respective peut être déterminée par recalage rigide dans le référentiel du système de navigation 30, ou dans le référentiel du robot médical 10, grâce à la position connue du marqueur sonde 41 relativement à la position des éléments émetteur-récepteur de la sonde à ultrasons 40.

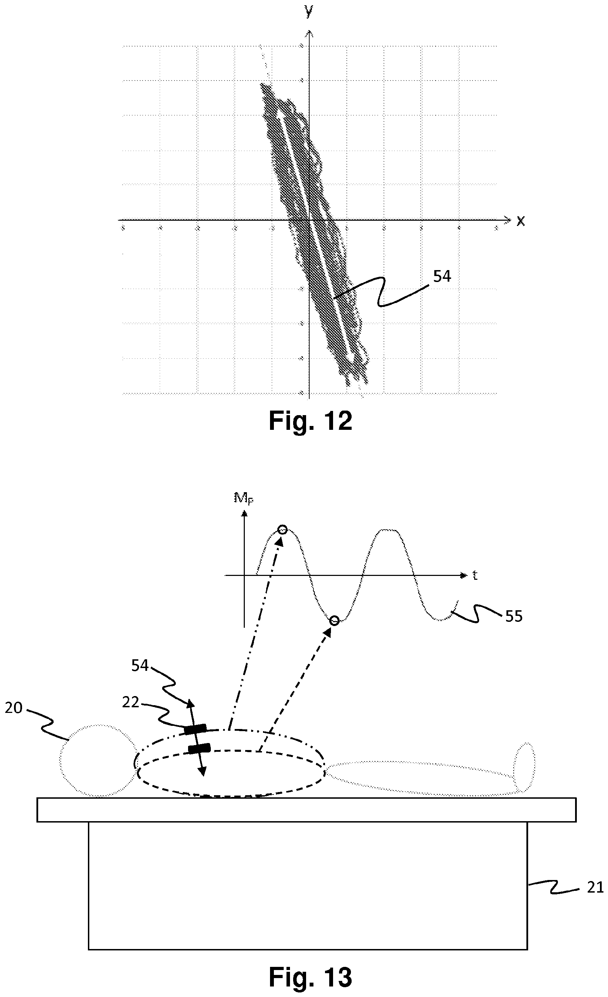

Les figures 12 à 14 illustrent l'étape de modélisation 207 de la position du point cible 51 et du point d'entrée 52 en fonction de la position du marqueur patient 22.

La figure 12 représente, à titre d'exemple, un enregistrement du mouvement suivi par le marqueur patient 22 pendant une période d'enregistrement d'une durée prédéterminée correspondant à plusieurs cycles respiratoires du patient. Chaque point correspond à une position prise par le marqueur patient 22 au cours du temps dans un plan XY d'un système de coordonnées du système de navigation 30 (le mouvement du marqueur patient 22 pourrait aussi être représenté dans le référentiel du robot médical 10). On constate, dans cet exemple, que le mouvement du marqueur patient 22 s'effectue principalement selon un axe 54.

Le mouvement du marqueur patient 22 est représentatif du mouvement de la cage thoracique du patient provoqué par la respiration du patient. Pour une meilleure interprétation du mouvement du marqueur, et par analogie au cycle respiratoire du patient, il est préférable d'obtenir une courbe à une dimension, illustrant le mouvement oscillatoire du marqueur dans le temps. Il existe différentes méthodes permettant d'obtenir cette courbe à une dimension. Il est par exemple envisageable de considérer que le mouvement du marqueur est majoritairement vertical, et par conséquent de ne considérer que l'axe Y. Cependant, dans ce cas, une partie de l'amplitude du mouvement du marqueur est perdue. Selon un autre exemple, il est envisageable d'effectuer une analyse en composantes principales des positions du marqueur. Les positions du marqueur peuvent notamment être affichées selon une composante principale correspondant à l'axe 54 principal du mouvement du marqueur.

La figure 13 représente une courbe 55 décrivant le mouvement du marqueur patient 22 au cours du temps, pendant la période d'enregistrement, selon l'axe 54 principal. La position (M_{P}) du marqueur patient 22 selon l'axe 54 est représentée en ordonnée ; le temps (t) est représenté en abscisse. La période d'enregistrement comporte plusieurs cycles respiratoires du patient 20.

Tel qu'illustré sur la figure 13, une position haute de la cage thoracique du patient 20 correspond à la fin d'une inspiration dans un cycle de respiration. Cela correspond aussi à un maximum de la courbe 55 décrivant la position du marqueur patient 22 au cours du temps. Une position basse de la cage thoracique du patient 20 correspond à la fin d'une expiration dans un cycle de respiration. Cela correspond aussi à un minimum de la courbe 55 décrivant la position du marqueur patient 22 au cours du temps.

Au cours de la phase de modélisation, l'unité de contrôle 12 détermine pour chaque image d'analyse la position du point cible, la position du point d'entrée et la position du marqueur patient. Ces positions peuvent alors être corrélées pour modéliser la position du point cible et la position du point d'entrée au cours du temps en fonction de la position du marqueur patient.

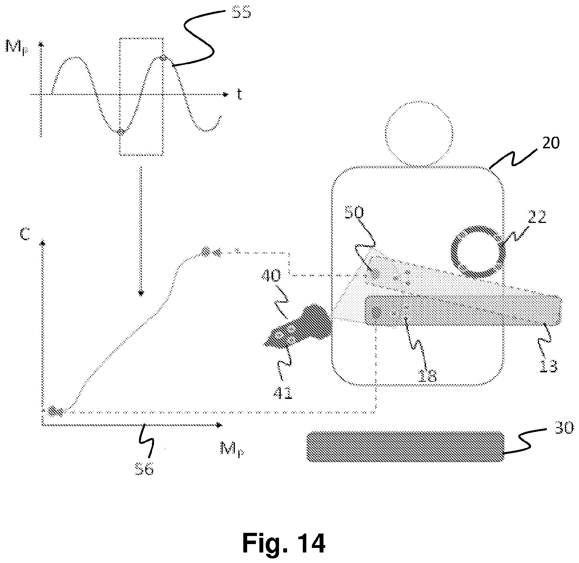

La figure 14 illustre, à titre d'exemple, un modèle 56 de la position du point cible (C) en fonction de la position du marqueur patient (M_{P}) pendant une période d'inspiration d'un cycle respiratoire.

Grâce à la modélisation de la position du point cible et de la position du point d'entrée au cours du temps en fonction de la position du marqueur patient, il devient possible de déterminer en temps réel la configuration que doit prendre le bras robotisé 13 pour que le guide-outil 14 soit constamment positionné de telle sorte que l'instrument médical 15 soit guidé par le guide-outil 14 selon la trajectoire définie par la position du point cible et la position du point d'entrée. Cet ajustement 303 en temps réel de la position du bras robotisé 13 peut être effectué simplement en suivant en temps réel la position du marqueur patient 22 et en utilisant le modèle permettant d'estimer la position du point cible et la position du point d'entrée en fonction de la position du marqueur patient. Il n'est alors plus nécessaire d'acquérir des images échographiques pour ajuster en temps réel la position du bras robotisé 13 pendant la phase de guidage.

Ainsi, il devient possible de bloquer la respiration du patient à n'importe quel instant du cycle respiratoire pour procéder à l'insertion de l'instrument médical. En effet, quel que soit l'instant où la respiration du patient est bloquée, le bras robotisé sera correctement positionné pour permettre l'insertion de l'instrument médical selon la trajectoire souhaitée.

Il n'est même plus indispensable de bloquer la respiration du patient pendant l'intervention. En effet, la phase de guidage du bras robotisé à l'aide du modèle peut être poursuivi pendant l'insertion de l'instrument médical. Le bras robotisé est alors contrôlé en temps réel pour que la position du bras robotisé soit constamment ajustée pour guider l'instrument médical selon la trajectoire souhaitée. Il convient toutefois de noter que pendant l'insertion de l'instrument médical, la position du point d'entrée au niveau de la peau du patient est fixe et devient un pivot de rotation pour les mouvements du bras robot.

La phase de guidage peut être réalisée en se basant entièrement sur le modèle généré pendant la phase de modélisation. Il n'est donc pas nécessaire que des images échographiques soient acquises pendant la phase de guidage.

Il peut toutefois être avantageux de prendre en compte un éventuel déplacement du point cible résultant de l'insertion de l'instrument médical. Le point cible peut en effet se déplacer dans la direction de la trajectoire suivie par l'instrument médical au cours de son insertion (c'est notamment le cas lorsque le point cible à atteindre est dans une lésion, par exemple une tumeur, au sein d'un organe mou). Il est alors envisageable de mettre à jour en temps réel le modèle permettant d'estimer la position du point cible afin d'ajuster la trajectoire que doit suivre l'instrument médical ainsi que la position du bras robotisé pour guider l'instrument médical selon cette trajectoire.

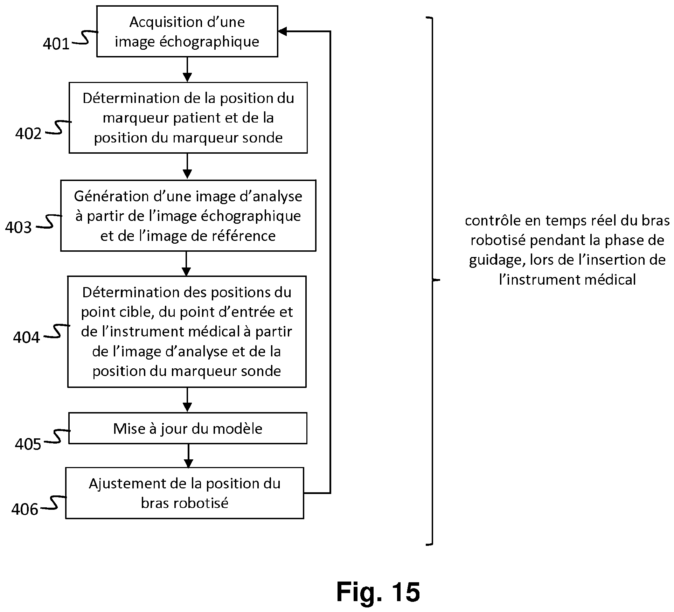

La figure 15 représente schématiquement les principales étapes d'un procédé mis en œuvre par l'unité de contrôle 12 pendant une phase de guidage au cours de l'insertion de l'instrument médical 15. L'unité de contrôle 12 est configurée pour recevoir de nouvelles images échographiques acquises par la sonde à ultrasons 40. Les étapes suivantes sont donc répétées pour chaque nouvelle image :
- acquisition 401 d'une image échographique,
- détermination 402 de la position du marqueur patient 22 et de la position du marqueur sonde 41 à l'instant auquel ladite image échographique a été acquise par la sonde à ultrasons 40,
- génération 403 d'une image d'analyse à partir de l'image échographique et de l'image de référence (à noter que cette étape est optionnelle si la lésion est directement visible sur l'image échographique),
- détermination 404, à partir de l'image d'analyse et de la position du marqueur sonde 41, de la position du point cible, de la position du point d'entrée, et de la position de l'instrument médical 15,
- mise à jour 405 du modèle initialement généré pendant la phase de modélisation,
- ajustement 406 de la position du bras robotisé 13 à partir du modèle mis à jour.

La détermination de la position de l'instrument médical pour chaque nouvelle image échographique reçue permet en outre de détecter une éventuelle courbure de l'instrument médical au cours de l'insertion, et le cas échéant d'ajuster le contrôle en temps réel du bras robotisé en conséquence.

Si la sonde à ultrasons 40 n'est pas correctement placée, il peut arriver que la lésion ne soit pas dans le champ de vue de la sonde à ultrasons. Il convient alors de pouvoir fournir au praticien une information sur une direction dans laquelle la sonde à ultrasons doit être déplacée pour que pour que la lésion soit dans le champ de vue de la sonde à ultrasons.

Dans ce but, l'unité de contrôle 12 peut être configurée pour comparer une image échographique avec une image de référence sur laquelle la lésion est visible, et pour donner une indication au praticien de la direction dans laquelle il convient de déplacer la sonde à ultrasons 40 pour qu'une image échographique acquise par la sonde à ultrasons 40 comporte une région anatomique dans laquelle se trouve la lésion. Cette indication peut par exemple être fournie par des indicateurs lumineux ou par un module de retour haptique de la sonde à ultrasons. Alternativement, cette indication peut être fournie au praticien via l'interface graphique 19.

La description ci-avant illustre clairement que, par leurs différentes caractéristiques et leurs avantages, les dispositifs et les méthodes présentés atteignent les objectifs fixés.

En particulier, le robot médical 10 utilise les informations de respiration pour positionner le guide-outil 14 de manière à suivre la position de la lésion avec précision et en temps réel, sans gestion spécifique par le praticien.

Au cours de l'insertion de l'instrument médical 15, la lésion (en particulier dans les organes mous) peut se déplacer dans la direction de la trajectoire. La détermination en temps réel de la position de la lésion sur les images échographiques et sa corrélation avec la position du marqueur patient 22 permettent de mettre à jour en temps réel la trajectoire de l'instrument médical 15 inséré par le bras robotisé. Les réajustements latéraux de trajectoire (qui sont généralement traumatique pour l'anatomie d'intérêt) sont minimisés. Le suivi de la position de l'instrument médical, au cours de son insertion, permet de compenser une éventuelle courbure de l'instrument médical.

Le contrôle en temps réel de la position du bras robotisé 13 est effectué grâce à des images échographiques. Le patient et le personnel soignant ne sont donc pas exposés à des rayonnements ionisants au cours de l'intervention.

Une lésion peu ou pas visible sur des images échographiques peut être détectée par recalage avec la modalité d'imagerie qui offre la meilleure visibilité.

## Revendications

1. Robot médical (10) pour assister un praticien lors d'une intervention médicale pour traiter une lésion dans une anatomie d'intérêt d'un patient (20), ledit robot médical (10) comportant un bras robotisé (13) comportant à une extrémité distale un guide-outil (14) destiné à guider un instrument médical (15), ainsi qu'une unité de contrôle (12) configurée pour contrôler le bras robotisé (13), le robot médical (10) étant configuré pour coopérer avec un système de navigation (30) et avec une sonde à ultrasons (40) destinée à être positionnée par le praticien au niveau de l'anatomie d'intérêt du patient, l'unité de contrôle (12) étant configurée pour pouvoir déterminer en tout instant, à partir d'informations communiquées par le système de navigation (30), la position d'un marqueur robot (18) destiné à être fixé sur le robot médical (10), la position d'un marqueur patient (22) destiné à être positionné sur le patient (20) à proximité de l'anatomie d'intérêt, et la position d'un marqueur sonde (41) destiné à être fixé sur la sonde à ultrasons (40), ledit robot médical (10) étant **caractérisé en ce que :**
- pendant une phase de modélisation, l'unité de contrôle (12) est configurée pour recevoir une pluralité d'images échographiques acquises par la sonde à ultrasons (40) pendant au moins un cycle respiratoire du patient et pour générer, à partir desdites images échographiques, un modèle permettant d'estimer la position d'un point cible au niveau de la lésion et la position d'un point d'entrée au niveau de la peau du patient (20) quel que soit l'instant considéré dans un cycle respiratoire ultérieur du patient, en fonction de la position du marqueur patient (22) audit instant considéré, sans qu'il soit nécessaire d'acquérir une image échographique audit instant considéré,
- pendant une phase de guidage, l'unité de contrôle (12) est configurée pour contrôler en temps réel le bras robotisé (13) en fonction de la position du marqueur patient (22) de sorte que le guide-outil (14) permette de guider l'instrument médical (15) selon une trajectoire définie par la position du point cible et la position du point d'entrée associées à la position du marqueur patient (22) dans le modèle.

2. Robot médical (10) selon la revendication 1 dans lequel, pendant la phase de modélisation, l'unité de contrôle (12) est configurée pour :
- pour chaque image échographique reçue :
∘ déterminer la position du marqueur patient (22) et la position du marqueur sonde (41) à l'instant auquel ladite image échographique a été acquise par la sonde à ultrasons (40),
∘ obtenir une image d'analyse, à partir de l'image échographique, sur laquelle la lésion est visible,
∘ déterminer, sur l'image d'analyse, un point cible au niveau de la lésion et un point d'entrée au niveau de la peau du patient (20), le point cible et le point d'entrée définissant ainsi une trajectoire à suivre pour l'instrument médical (15),
∘ déterminer la position du point cible et la position du point d'entrée à partir de la position du marqueur sonde (41),
∘ établir une association entre la position du marqueur patient (22), la position du point cible et la position du point d'entrée ainsi déterminées pour ladite image échographique,
- modéliser, à partir des informations ainsi obtenues pour la pluralité d'images échographiques, la position du point cible et la position du point d'entrée, en fonction de la position du marqueur patient (22), quel que soit l'instant considéré dans un cycle respiratoire du patient.

3. Robot médical (10) selon l'une quelconque des revendications 1 à 2 dans lequel, pendant la phase de guidage, lors d'une insertion de l'instrument médical (15), l'unité de contrôle (12) est configurée pour recevoir régulièrement de nouvelles images échographiques acquises par la sonde à ultrasons (40) et pour mettre à jour le modèle à partir desdites nouvelles images échographiques.

4. Robot médical (10) selon la revendication 3 dans lequel pendant la phase de guidage, lors de l'insertion de l'instrument médical (15), l'unité de contrôle (12) est configurée pour déterminer, pour chaque nouvelle image échographique reçue, la position de l'instrument médical (15), et pour ajuster le contrôle en temps réel du bras robotisé (13) en fonction de la position de l'instrument médical (15).

5. Robot médical (10) selon l'une quelconque des revendications 2 à 4 dans lequel l'image d'analyse est l'image échographique, la lésion étant visible sur l'image échographique.

6. Robot médical (10) selon l'une quelconque des revendications 2 à 4 dans lequel l'unité de contrôle est configurée pour obtenir l'image d'analyse en fusionnant l'image échographique avec une image de référence préopératoire ou pré-interventionnelle sur laquelle la lésion est visible.

7. Robot médical (10) selon la revendication 6 dans lequel un élément radio-opaque (24) du marqueur patient (22) est visible sur l'image de référence, et l'unité de contrôle (12) est configurée pour fusionner l'image échographique avec l'image de référence par un recalage basé sur la position du marqueur patient (22) relativement à la position du marqueur sonde (41) à l'instant auquel l'image échographique a été acquise par la sonde à ultrasons (40).

8. Robot médical (10) selon l'une quelconque des revendications 6 à 7 dans lequel l'image de référence est une image de tomodensitométrie, une image de tomographie par émission de positons ou une image d'imagerie par résonance magnétique.

9. Robot médical (10) selon l'une quelconque des revendications 1 à 8 dans lequel les images échographiques reçues de la sonde à ultrasons (40) sont des images échographiques en mode B.

10. Robot médical (10) selon l'une quelconque des revendications 1 à 9 dans lequel l'unité de contrôle (12) est configurée pour recevoir et traiter des images échographiques acquises par la sonde à ultrasons (40) à une fréquence au moins égale à quinze images par seconde.

11. Robot médical (10) selon l'une quelconque des revendications 2 à 10 comportant une interface utilisateur (19) comprenant un écran d'affichage permettant au praticien de visualiser les images d'analyse.

12. Robot médical (10) selon la revendication 11 dans lequel l'interface utilisateur (19) comporte des moyens d'entrée permettant au praticien d'identifier sur une image d'analyse affichée sur l'écran d'affichage un point cible et/ou un point d'entrée et/ou une zone anatomique qui ne doit pas être traversée par l'instrument médical (15).

13. Robot médical (10) selon l'une quelconque des revendications 11 à 12 dans lequel l'interface utilisateur comprend un dispositif de réalité augmentée permettant de superposer les images d'analyse avec des images réelles du corps du patient sur l'écran d'affichage.

14. Robot médical (10) selon l'une quelconque des revendications 1 à 13 dans lequel l'unité de contrôle (12) est configurée pour comparer une image échographique avec une image de référence sur laquelle la lésion est visible, et pour donner une indication au praticien de la direction dans laquelle il convient de déplacer la sonde à ultrasons (40) pour qu'une image échographique acquise par la sonde à ultrasons (40) comporte une région anatomique dans laquelle se trouve la lésion.

15. Dispositif médical comportant un robot médical (10) selon l'une des revendications 1 à 14, ainsi qu'un système de navigation (30) et une sonde à ultrasons (40) destinés à coopérer avec le robot médical (10).

## Patentansprüche

1. Medizinischer Roboter (10) zur Unterstützung eines Arztes während eines medizinischen Eingriffs zur Behandlung einer Läsion in einer Anatomie von Interesse eines Patienten (20), wobei der medizinische Roboter (10) einen Roboterarm (13) umfasst, der an einem distalen Ende eine Werkzeugführung (14), die zur Führung eines medizinischen Instruments (15) bestimmt ist, sowie eine Steuereinheit (12), die zum Steuern des Roboterarms (13) konfiguriert ist, umfasst, wobei der medizinische Roboter (10) zur Zusammenarbeit mit einem Navigationssystem (30) und mit einer Ultraschallsonde (40) konfiguriert ist, die zum Positionieren durch den Arzt auf Höhe der Anatomie von Interesse des Patienten bestimmt ist, wobei die Steuereinheit (12) konfiguriert ist, um jederzeit anhand der vom Navigationssystem (30) kommunizierten Informationen die Position einer Robotermarkierung (18), die zum Befestigen auf dem medizinischen Roboter (10) bestimmt ist, die Position einer Patientenmarkierung (22), die zum Positionieren auf dem Patienten (20) in der Nähe der Anatomie von Interesse bestimmt ist, und die Position einer Sondenmarkierung (41), die zum Befestigen auf der Ultraschallsonde (40) bestimmt ist, bestimmen zu können, wobei der medizinische Roboter (10) **dadurch gekennzeichnet ist, dass:**
- während einer Modellierungsphase die Steuereinheit (12) konfiguriert ist, um eine Vielzahl von Ultraschallbildern zu empfangen, die von der Ultraschallsonde (40) während mindestens eines Atemzyklus des Patienten erfasst werden, und aus diesen Ultraschallbildern ein Modell zu generieren, das es ermöglicht, die Position eines Zielpunkts auf Höhe der Läsion und die Position eines Eintrittspunkts auf Höhe der Haut des Patienten (20) abzuschätzen, unabhängig vom betrachteten Zeitpunkt in einem nachträglichen Atemzyklus des Patienten, in Abhängigheit von der Position der Patientenmarkierung (22) zu diesem betrachteten Zeitpunkt, ohne dass es erforderlich ist, zu diesem betrachteten Zeitpunkt ein Ultraschallbild zu erfassen,
- während einer Führungsphase die Steuereinheit (12) konfiguriert ist, um den Roboterarm (13) in Echtzeit in Abhängigkeit von der Position der Patientenmarkierung (22) so zu steuern, dass die Werkzeugführung (14) eine **Führung** des medizinisches Instruments (15) gemäß einer Trajektorie ermöglicht, die durch die Position des Zielpunkts und die Position des Eintrittspunkts definiert ist, die der Position der Patientenmarkierung (22) im Modell zugeordnet ist.

2. Medizinischer Roboter (10) nach Anspruch 1, bei dem die Steuereinheit (12) während der Modellierungsphase konfiguriert ist zum:
- für jedes empfangene Ultraschallbild:
o Bestimmen der Position der Patientenmarkierung (22) und der Position der Sondenmarkierung (41) zu dem Zeitpunkt, zu dem das Ultraschallbild von der Ultraschallsonde (40) erfasst wurde,
o Erhalten eines Analysebildes aus dem Ultraschallbild, auf dem die Läsion sichtbar ist,
o Bestimmen, auf dem Analysebild, eines Zielpunkts auf Höhe der Läsion und eines Eintrittspunkts auf Höhe der Haut des Patienten (20), wobei der Zielpunkt und der Eintrittspunkt so eine Trajektorie definieren, der das medizinische Instrument (15) folgen soll,
o Bestimmen der Position des Zielpunkts und der Position des Eintrittspunkts aus der Position der Sondenmarkierung (41),
o Herstellen einer Zuordnung zwischen der so bestimmten Position der Patientenmarkierung (22), der Position des Zielpunkts und der Position des Eintrittspunkts für das Ultraschallbild,
- Modellieren, aus den so für die Vielzahl von Ultraschallbildern erhaltenen Informationen, der Position des Zielpunkts und der Position des Eintrittspunkts in Abhängigkeit von der Position der Patientenmarkierung (22), unabhängig vom betrachteten Zeitpunkt in einem Atemzyklus des Patienten.

3. Medizinischer Roboter (10) nach einem der Ansprüche 1 bis 2, bei dem die Steuereinheit (12) während der Führungsphase während des Einführens des medizinischen Instruments (15) konfiguriert ist, um regelmäßig neue Ultraschallbilder zu empfangen, die durch die Ultraschallsonde (40) erfasst werden, und das Modell anhand der neuen Ultraschallbilder zu aktualisieren.

4. Medizinischer Roboter (10) nach Anspruch 3, bei dem während der Führungsphase während des Einführens des medizinischen Instruments (15) die Steuereinheit (12) konfiguriert ist, um für jedes neue empfangene Ultraschallbild die Position des medizinischen Instruments (15) zu bestimmen und um die Echtzeitsteuerung des Roboterarms (13) in Abhängigkeit von der Position des medizinischen Instruments (15) anzupassen.

5. Medizinischer Roboter (10) nach einem der Ansprüche 2 bis 4, bei dem das Analysebild das Ultraschallbild ist, wobei die Läsion auf dem Ultraschallbild sichtbar ist.

6. Medizinischer Roboter (10) nach einem der Ansprüche 2 bis 4, bei dem die Steuereinheit konfiguriert ist, um das Analysebild durch Zusammenführen des Ultraschallbilds mit einem präoperativen oder präinterventionellen Referenzbild zu erhalten, auf dem die Läsion sichtbar ist.

7. Medizinischer Roboter (10) nach Anspruch 6, bei dem ein röntgendichtes Element (24) der Patientenmarkierung (22) auf dem Referenzbild sichtbar ist, und die Steuereinheit (12) konfiguriert ist, um das Ultraschallbild mit dem Referenzbild durch Neueinstellung basierend auf der Position der Patientenmarkierung (22) relativ zur Position der Sondenmarkierung (41) zu dem Zeitpunkt, zu dem das Ultraschallbild von der Ultraschallsonde (40) erfasst wurde, zusammenzuführen.

8. Medizinischer Roboter (10) nach einem der Ansprüche 6 bis 7, wobei das Referenzbild ein Computertomographiebild, ein Positronen-Emissions-Tomographiebild oder ein Magnetresonanztomographiebild ist.

9. Medizinischer Roboter (10) nach einem der Ansprüche 1 bis 8, bei dem die von der Ultraschallsonde (40) empfangenen Ultraschallbilder B-Mode-Ultraschallbilder sind.

10. Medizinischer Roboter (10) nach einem der Ansprüche 1 bis 9, bei dem die Steuereinheit (12) konfiguriert ist, um von der Ultraschallsonde (40) erfasste Ultraschallbilder mit einer Frequenz von mindestens fünfzehn Bildern pro Sekunde zu empfangen und zu verarbeiten.

11. Medizinischer Roboter (10) nach einem der Ansprüche 2 bis 10, umfassend eine Benutzerschnittstelle (19), die einen Anzeigebildschirm umfasst, der es dem Arzt ermöglicht, die Analysebilder anzusehen.

12. Medizinischer Roboter (10) nach Anspruch 11, bei dem die Benutzerschnittstelle (19) Eintrittsmittel umfasst, die es dem Arzt ermöglichen, auf einem auf dem Anzeigebildschirm angezeigten Analysebild einen Zielpunkt und/oder einen Eintrittspunkt und/oder eine anatomische Zone zu identifizieren, die vom medizinischen Instrument (15) nicht überschritten werden darf.

13. Medizinischer Roboter (10) nach einem der Ansprüche 11 bis 12, bei dem die Benutzerschnittstelle eine Augmented-Reality-Vorrichtung umfasst, die es ermöglicht, die Analysebilder mit realen Bildern des Körpers des Patienten auf dem Anzeigebildschirm zu überlagern.

14. Medizinischer Roboter (10) nach einem der Ansprüche 1 bis 13, bei dem die Steuereinheit (12) konfiguriert ist, um ein Ultraschallbild mit einem Referenzbild zu vergleichen, auf dem die Läsion sichtbar ist, und dem Arzt einen Hinweis zur Richtung zu geben, in der die Ultraschallsonde (40) bewegt werden soll, damit ein von der Ultraschallsonde (40) aufgenommenes Ultraschallbild einen anatomischen Bereich umfasst, in dem sich die Läsion befindet.

15. Medizinische Vorrichtung, umfassend einen medizinischen Roboter (10) nach einem der Ansprüche 1 bis 14 sowie ein Navigationssystem (30) und eine Ultraschallsonde (40) zur Zusammenarbeit mit dem medizinischen Roboter (10).

## Claims

1. A medical robot (10) for assisting a practitioner during a medical intervention for treating a lesion in an anatomy of interest of a patient (20), said medical robot (10) comprising a robotic arm (13) comprising, at a distal end, a tool guide (14) intended to guide a medical instrument (15), and a control unit (12) configured to control the robotic arm (13), the medical robot (10) being configured to cooperate with a navigation system (30) and with an ultrasound probe (40) to be positioned by the practitioner at the anatomy of interest of the patient, the control unit (12) being configured to be able to determine at any time, based on information communicated by the navigation system (30), the position of a robot marker (18) to be attached to the medical robot (10), the position of a patient marker (22) to be positioned on the patient (20) proximate to the anatomy of interest, and the position of a probe marker (41) to be attached to the ultrasound probe (40), said medical robot (10) being **characterized in that:**
- during a modeling phase, the control unit (12) is configured to receive a plurality of ultrasound images acquired by the ultrasound probe (40) during at least one respiratory cycle of the patient and to generate, from said ultrasound images, a model for estimating the position of a target point at the lesion and the position of an entry point at the skin of the patient (20) irrespective of the moment considered in a subsequent respiratory cycle of the patient, based on the position of the patient marker (22) at said considered moment, without it being necessary to acquire an ultrasound image at said considered moment,
- during a guidance phase, the control unit (12) is configured to control the robotic arm (13) in real time based on the position of the patient marker (22) such that the tool guide (14) allows guiding of the medical instrument (15) along a trajectory defined by the position of the target point and the position of the entry point associated with the position of the patient marker (22) in the model.

2. The medical robot (10) according to claim 1, wherein, during the modeling phase, the control unit (12) is configured to:
- for each ultrasound image received:
∘ determine the position of the patient marker (22) and the position of the probe marker (41) at the moment that said ultrasound image was acquired by the ultrasound probe (40),
∘ obtain an analysis image, from the ultrasound image, on which the lesion is visible,
∘ determine, on the analysis image, a target point at the lesion and an entry point at the skin of the patient (20), the target point and the entry point thus defining a trajectory to be followed for the medical instrument (15),
∘ determine the position of the target point and the position of the entry point from the position of the probe marker (41),
∘ establish an association between the position of the patient marker (22), the position of the target point and the position of the entry point thus determined for said ultrasound image,
- to model, from the information thus obtained for the plurality of ultrasound images, the position of the target point and the position of the entry point, based on the position of the patient marker (22), irrespective of the moment considered in a respiratory cycle of the patient.

3. The medical robot (10) according to any one of claims 1 to 2, wherein, during the guiding phase, upon insertion of the medical instrument (15), the control unit (12) is configured to regularly receive new ultrasound images acquired by the ultrasound probe (40) and to update the model based on said new ultrasound images.

4. The medical robot (10) according to claim 3, wherein, during the guiding phase, upon insertion of the medical instrument (15), the control unit (12) is configured to determine, for each new received ultrasound image, the position of the medical instrument (15), and to adjust the real-time control of the robotic arm (13) based on the position of the medical instrument (15).

5. The medical robot (10) according to any one of claims 2 to 4, wherein the analysis image is the ultrasound image, the lesion being visible on the ultrasound image.

6. The medical robot (10) according to any one of claims 2 to 4, wherein the control unit is configured to obtain the analysis image by merging the ultrasound image with a pre-operative or pre-interventional reference image on which the lesion is visible.

7. The medical robot (10) according to claim 6, wherein a radiopaque element (24) of the patient marker (22) is visible on the reference image, and the control unit (12) is configured to merge the ultrasound image with the reference image by registration based on the position of the patient marker (22) relative to the position of the probe marker (41) at the moment when the ultrasound image was acquired by the ultrasound probe (40).

8. The medical robot (10) according to any one of claims 6 to 7, wherein the reference image is a computed tomography image, a positron emission tomography image or a magnetic resonance imaging image.

9. The medical robot (10) according to any one of claims 1 to 8, wherein the ultrasound images received from the ultrasound probe (40) are B-mode ultrasound images.

10. The medical robot (10) according to any one of claims 1 to 9, wherein the control unit (12) is configured to receive and process ultrasound images acquired by the ultrasound probe (40) at a rate of at least fifteen images per second.

11. The medical robot (10) according to any one of claims 2 to 10, comprising a user interface (19) comprising a display screen enabling the practitioner to view the analysis images.

12. The medical robot (10) according to claim 11, wherein the user interface (19) comprises input means enabling the practitioner to identify, on an analysis image displayed on the display screen, a target point and/or an entry point and/or an anatomical region that is not to be traversed by the medical instrument (15).

13. The medical robot (10) according to any one of claims 11 to 12, wherein the user interface comprises an augmented reality device for superimposing the analysis images with actual images of the patient's body on the display screen.

14. The medical robot (10) according to any one of claims 1 to 13, wherein the control unit (12) is configured to compare an ultrasound image with a reference image on which the lesion is visible, and to give an indication to the practitioner of the direction in which the ultrasound probe (40) should be moved so that an ultrasound image acquired by the ultrasound probe (40) comprises an anatomical region wherein the lesion is located.

15. A medical device comprising a medical robot (10) according to one of claims 1 to 14, and a navigation system (30) and an ultrasound probe (40) for cooperating with the medical robot (10).
